# EUROPÄISCHE PATENTSCHRIFT

(11) **EP 2 848 235 B1**
(45) Veröffentlichungstag und Bekanntmachung des Hinweises auf die Patenterteilung: **02.05.2018**
(21) Anmeldenummer: 14184216.1
(22) Anmeldetag: 10.09.2014
(51) Int. Cl.: A61F 13/08, D04B 1/18, D04B 1/26, D04B 1/28, D04B 7/00

(54) **Kompressionsartikel**
Compression article
Article de compression

(30) Priorität: 13.09.2013 DE 102013218420
(43) Veröffentlichungstag der Anmeldung: 18.03.2015
(73) Patentinhaber: BSN -Jobst GmbH, 46446 Emmerich (DE)
(72) Erfinder: Platz, Sascha, 47533 Kleve (DE); Greve, Jürgen, 46446 Emmerich (DE)
(74) Vertreter: Kohler Schmid Möbus Patentanwälte

(56) Entgegenhaltungen:
- EP-A1- 1 679 012
- EP-A1- 2 792 774
- WO-A1-2007/055239
- DE-A1- 19 616 003
- BLAGA ET AL: "gestrickte fingerhandschuhe als Zusatzsortiment", MELLIAND TEXTILBERICHTE, DEUTSCHER FACHVERLAG, FRANKFURT AM MAIN, DE, Bd. 90, Nr. 3, 1. Januar 2009 (2009-01-01) , Seiten 94-95, XP001526569, ISSN: 0341-0781

## Beschreibung

Die Erfindung betrifft einen Kompressionsartikel mit einem Hauptschlauchgestrick, in das mindestens zwei Teilschlauchgestricke münden, wobei das Haupt- und die Teilschlauchgestricke auf einer Flachstrickmaschine nahtlos hergestellt sind.

Solche Kompressionsartikel sind bereits aus der DE 10 2004 036 344 bekannt. Sie können beispielsweise als Handschuhe oder Zehensocken oder -strümpfe ausgebildet sein und dienen in erster Linie der Behandlung von Lymphödemen.

Für eine erfolgreiche Therapie ist es entscheidend, dass der Kompressionsartikel an allen Stellen einen definierten Kompressionsdruckverlauf auf den Körper des Patienten ausübt. Eine optimale Anpassung des Artikels an die Gestalt der Gliedmaßen des Patienten ist daher unabdingbar.

Bei Handschuhen oder Zehensocken ist die Herstellung einer optimalen Passform und das Aufrechterhalten eines definierten Kompressionsdruckverlaufs am Übergang von den Finger- oder Zehenröhren zur Hand- oder Fußröhre am schwierigsten, da die Summe der Maschen der die Finger oder Zehen umschließenden Schlauchgestricke größer ist als die Maschenzahl des den Handteil oder den Fußteil umschließenden Schlauchgestricks.

Zur Lösung dieses Problems ist in der DE 10 2007 063 148 A1 ein Verfahren beschrieben, nach dem die überschüssigen Maschen der Teilschlauchgestricke erst überdeckt und dann in einer Maschenreihe abgekettelt werden. Dadurch ist zwar eine gute Passform des Hand- oder Fußteils erreichbar, doch entstehen durch die Überdeckung der Maschen der Teilschlauchgestricke der Finger- oder Zehenröhren leichte Verdrehungen, die den Trägerkomfort beeinträchtigen können. Ebenso entstehen durch das Abketteln zwischen den Finger- oder Zehenröhren Spannungen, welche ebenfalls den Tragekomfort oder die Funktion des Kompressionsartikels beeinträchtigen können.

Die Druckschrift DE 196 15 003 A1 beschreibt die dreidimensionale Formungsmöglichkeit von Gestricken durch Spickeln, Mindern und Zunehmen von Maschen. Insbesondere wird die Anwendung auf Überzüge für Sitzmöbel beschrieben.

In der EP 1 679 012 A1 wird ein Handschuh beschrieben, bei dem der Druck in den Fingerkörpern und im Vierfingerteil, d.h. dem Handteil, sowie in den Übergangsbereichen zwischen den Fingerkörpern und dem Vierfingerkörper durch die Variation der Vorspannung eines Schussfadens und die Variation der Maschenlänge eingestellt wird.

Im Artikel Blage et a.: Gestrickte Fingerhandschuhe als Zusatzsortiment", Melliand Textilberichte, Deutscher Fachverlag, Frankfurt am Main, Bd. 90, Nr. 3, 1. Januar 2009, Seiten 94-95 ist die Herstellung eines modischen Fünffingerhandschuhs ohne Maschenminderungszone im Übergangsbereich zwischen den Fingern und dem Handteil beschrieben. Bei dem in der Druckschrift WO 2007/055239 A1 beschriebenen Handschuh findet eine Minderung der Maschen zwischen den Fingern und dem Handteil in einer einzigen Maschenreihe und in nur einer der Gestricklagen statt. Die Druckschrift EP 2 792 774 A1 offenbart bindungs- und materialtechnische Gestaltungen eines Schlauchgestricks, nicht jedoch das Verbinden mehrerer Schlauchgestricke.

Der vorliegenden Erfindung liegt die Aufgabe zugrunde, einen Kompressionsartikel der eingangs genannten Art mit gleichmäßigen Kompressionseigenschaften und einer verbesserten Passform sowie ein Verfahren zu seiner Herstellung bereitzustellen.

Die Aufgabe wird gelöst durch einen Kompressionsartikel mit einem Hauptschlauchgestrick, in das mindestens zwei Teilschlauchgestricke münden, wobei das Haupt- und die Teilschlauchgestricke auf einer Flachstrickmaschine nahtlos hergestellt sind, der dadurch gekennzeichnet ist, dass im Bereich des Übergangs zwischen den Teilschlauchgestricken und dem Hauptschlauchgestrick eine sich über mehrere Maschenreihen erstreckende Maschenminderungszone an den Teilgestricken vorhanden ist.

Durch diese Maschenminderungszonen entstehen Spickel zwischen den Teilschlauchgestricken und dem Hauptschlauchgestrick, mit deren Hilfe Spannungen in den Teilgestricken oder ein Verdrehen der Teilgestricke vermieden werden können. Dadurch verbessert sich der Tragekomfort der Kompressionsartikel im Vergleich zu bekannten Artikeln. Dennoch bleiben die Kompressionseigenschaften konstant.

Bevorzugt können die Maschenminderungen mindestens an einer zwischen vorderer und hinterer Gestricklage angeordneten Umfangsstelle der Teilschlauchgestricke angeordnet sein. Bei Handschuhen oder Zehenstrümpfen liegen die Minderungsstellen dadurch symmetrisch zwischen den Fingern oder Zehen.

Müssen aufgrund der anatomischen Eigenschaften des Patienten relativ viele Maschen zwischen den Teilschlauchgestricken und dem Hauptschlauchgestrick gemindert werden, so können die Teilschlauchgestricke über ihren Umfang verteilt mehrere Maschenminderungsstellen aufweisen. Dadurch lassen sich in der gleichen Anzahl von Maschenreihen mehr Maschen an den Teilschlauchgestricken mindern.

Eine weitere Verbesserung des Tragekomforts lässt sich dadurch erzielen, dass sich die Teilschlauchgestricke im Übergangsbereich zum Hauptschlauchgestrick teilweise überlappen. Die Teilschlauchgestricke liegen damit leicht schuppenförmig übereinander, wodurch der Tragekomfort im Bereich zwischen den Zehen oder Fingern erhöht wird.

Auch außerhalb des Übergangsbereichs zwischen den Teil- und dem Hauptschlauchgestrick ist das Kompressionsgestrick zweckmäßigerweise der Form des zu stützenden Körperteils durch entsprechende Durchmesseränderungen der Schlauchgestricke anatomisch angepasst gestrickt, um überall die gewünschte gleichmäßige Kompressionswirkung sicherstellen zu können.

Weiter kann der Kompressionsartikel in an sich bekannter Weise unter Verwendung mindestens eines elastischen Schussfadens hergestellt sein. Über die Elastizität des oder der Schussfäden und die Art ihrer Einbindung in das Gestrick lässt sich der Kompressionsdruck sehr präzise einstellen.

Die Erfindung betrifft außerdem ein Verfahren zur Herstellung eines erfindungsgemäßen Kompressionsartikels auf einer Flachstrickmaschine, das dadurch gekennzeichnet ist, dass am Übergang von den Teilschlauchgestricken zum Hauptschlauchgestrick einige der Maschen benachbarter Teilschlauchgestricke auf gemeinsame Nadeln gehängt und in kurzen Zwischenmaschenreihen abgestrickt werden, bevor die Maschenzahl der Teilschlauchgestricke durch Umhängevorgänge und Nadelbettenversatzbewegungen über mehrere Maschenreihen hinweg gemindert werden.

Die kurzen Zwischenmaschenreihen führen zu einer Maschenanhäufung in diesen Bereichen zwischen den Teilschlauchgestricken, die zu einer Reduktion der Spannung auf die Teilgestricke durch die Minderungsvorgänge führen. Der Kompressionsartikel wird dadurch gleichmäßiger gestrickt und erhält einen verbesserten Tragekomfort.

Weitere Vorteile ergeben sich, wenn die Umhängevorgänge und Versatzbewegungen mit Hilfe von Zusatznadelbetten durchgeführt werden. Dadurch müssen keine Einschränkungen hinsichtlich der Grundbindung für die Schlauchgestricke in Kauf genommen werden. Außerdem sorgen Umhängevorgänge auf Zusatznadelbetten für eine geringere Belastung der Maschen als ein Umhängen auf das benachbarte Nadelbett.

Bei einer bevorzugten Verfahrensvariante werden die Teilschlauchgestricke und das Hauptschlauchgestrick jeweils in einer Rippbindung mit einem durchgehenden Schussfaden hergestellt. Rippbindungen weisen eine hohe Elastizität auf und sorgen damit für einen hohen Tragekomfort. Durch den verwendeten Schussfaden und die Art seiner Einbindung kann der Kompressionsdruck des Artikels eingestellt werden.

Dabei kann die Rippbindung vorzugsweise mit einem unelastischen Faden (z. B. Polyamid, Polyester) und einem Elastikfaden hergestellt werden. Der unelastische Faden verleiht dem Gestrick die notwendige Festigkeit.

Einzelne Maschenreihen der Rippbindung können auch ausschließlich mit einem unelastischen Faden hergestellt werden. Insbesondere kann mit diesen Maschenreihen der Schussfaden in das Gestrick eingebunden werden.

Nachfolgend wird ein bevorzugtes Ausführungsbeispiel eines erfindungsgemäßen Verfahrens anhand der Anbindung von Fingerschlauchgestricken an ein Handschlauchgestrick im Einzelnen beschrieben.

Es zeigen:
- Fig. 1: eine schematische Darstellung der Grundbindung der Schlauchgestricke;
- Fig. 2: eine schematische Darstellung der Herstellung des Übergangsbereichs zwischen zwei Fingerschlauchgestricken und dem Handschlauchgestrick mit Minderung des ersten Fingerschlauchgestricks,
- Fig. 3: eine schematische Darstellung der Anbindung eines Daumenschlauchgestricks an das Handschlauchgestrick.

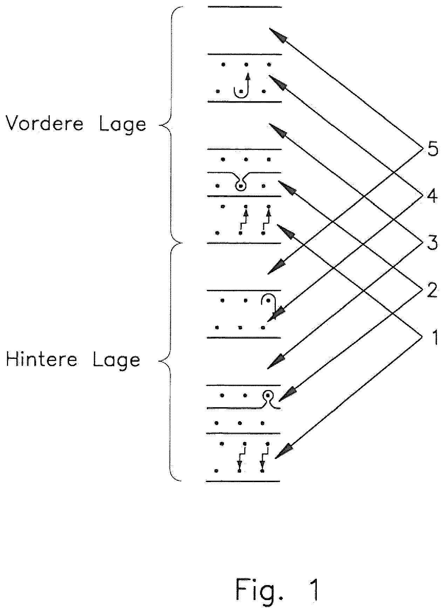

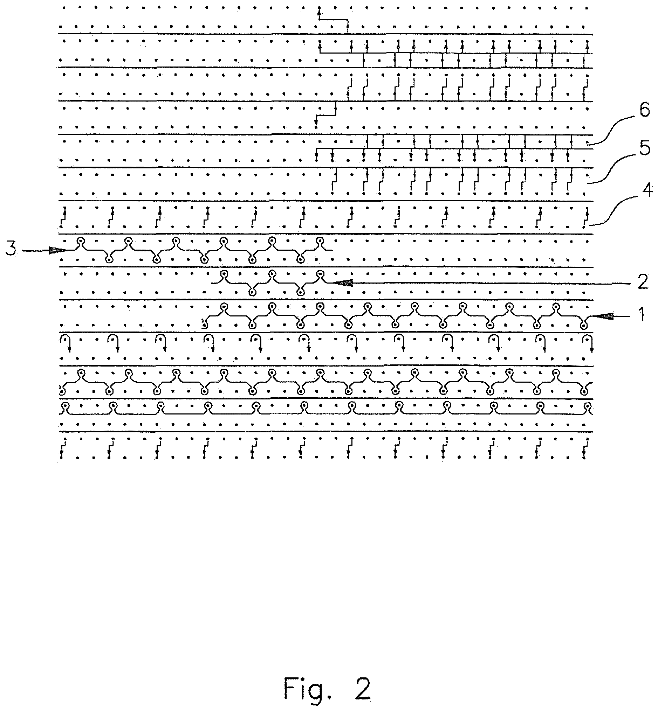

Zur Herstellung der in Fig. 1 gezeigten Grundbindung werden für die hintere Lage der Schlauchgestricke auf dem hinteren Nadelbett einer Flachstrickmaschine und für die vordere Lage auf dem vorderen Nadelbett von unten nach oben in Schritt 1 zunächst die Maschen für die jeweilige Gestricklage auf die Nadeln des jeweiligen Nadelbetts eingeteilt. Anschließend wird in Schritt 2 ein elastischer Schussfaden jeweils in leere Nadeln der beiden Nadelbetten eingelegt. Dieser Schussfaden wird in Schritt 3 in einer Strickreihe mit einem Polyamid-Faden fixiert, bevor er in Schritt 4 von den Nadeln abgeworfen wird. Anschließend folgt in Schritt 5 eine Strickreihe mit einem Elastikgarn und einem nicht elastischen Strickfaden, wofür auch der Polyamid-Faden aus Schritt 3 eingesetzt werden kann. In dieser plattierten Strickreihe können Minderungen vorgenommen werden, wie beispielsweise in Fig. 2 gezeigt ist.

Fig. 2 zeigt die Minderung eines rechten Fingers im Bereich der Verbindung mit einem linken Finger. In Schritt 1 wird eine plattierte Strickreihe für den rechten Finger ausgeübt, dann in Schritt 2 eine kurze plattierte Zwischenstrickreihe auf Nadeln am linken Rand des rechten Fingers gebildet, bevor in Schritt 3 eine plattierte Maschenreihe für einen linken Finger gebildet wird, wobei die Maschen am rechten Rand des linken Fingers auf die Nadeln mit den Maschen am linken Rand des rechten Fingers zur Verbindung der beiden Finger gehängt wurden. Anschließend werden in Schritt 4 und 5 die Maschen der vorderen Lage auf Nadeln eines Zusatznadelbetts umgehängt und dann die Nadelbetten beispielsweise um drei Maschen nach links versetzt. In Schritt 6 werden die Maschen der vorderen Lage wieder auf das vordere Nadelbett zurückgehängt. Die Umhängevorgänge und Versatzbewegungen werden anschließend in analoger Weise für die hintere Gestricklage ausgeführt. Danach ist der rechte Finger an seiner linken Seite um drei Maschen gemindert worden. Dieser Vorgang kann wiederholt werden. Es ist aber auch möglich, Minderungen des rechten Fingers an mehreren Stellen seines Umfangs durchzuführen, nicht nur an seiner linken Seite.

Sobald die Minderung des rechten Fingers abgeschlossen ist, wird der linke Finger an seiner rechten Seite in analoger Weise durch Umhängevorgänge und Nadelbettenversatzbewegungen gemindert.

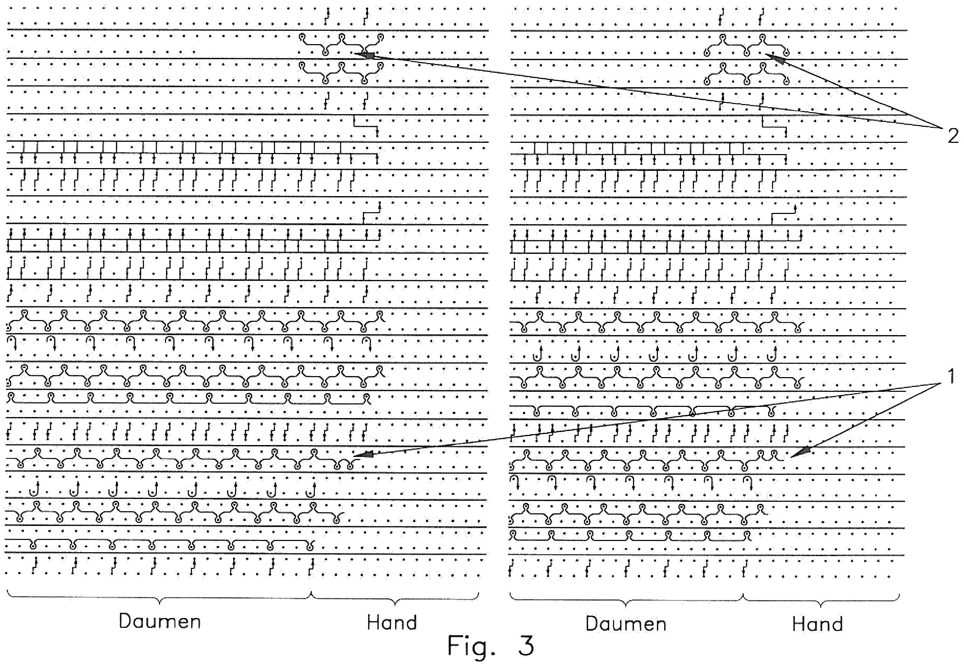

Fig. 3 illustriert das Anbinden eines Daumenschlauchgestricks an ein Handschlauchgestrick. Das Handschlauchgestrick wird abgekettelt, während das Daumenschlauchgestrick als Halbschlauch eingebunden wird.

In Schritt 1 werden beim Eindecken des Daumens auf Nadeln des Handbereichs nur Maschenreihen für den Daumen gestrickt. Das Eindecken erfolgt wieder über Umhängevorgänge und Nadelbettenversatzbewegungen mit Hilfe der Zusatznadelbetten.

Zusätzlich werden in Schritt 2 zwischen dem Daumen und der Hand kurze Extra-Maschenreihen gestrickt, um die Spannung zwischen Daumen und Handteil weiter zu reduzieren.

## Patentansprüche

1. Kompressionsartikel mit einem Hauptschlauchgestrick, in das mindestens zwei Teilschlauchgestricke münden, wobei das Haupt- und die Teilschlauchgestricke auf einer Flachstrickmaschine nahtlos hergestellt sind, **dadurch gekennzeichnet, dass** im Bereich des Übergangs zwischen den Teilschlauchgestricken und dem Hauptschlauchgestrick eine sich über mehrere Maschenreihen erstreckende Maschenminderungszone an den Teilgestricken vorhanden ist.

2. Kompressionsartikel nach Anspruch 1, **dadurch gekennzeichnet, dass** die Maschenminderungen mindestens an einer zwischen vorderer und hinterer Gestricklage angeordneten Umfangsstelle der Teilschlauchgestricke angeordnet sind.

3. Kompressionsartikel nach Anspruch 1 oder 2, **dadurch gekennzeichnet, dass** die Teilschlauchgestricke über ihren Umfang verteilt mehrere Maschenminderungsstellen aufweisen.

4. Kompressionsgestrick nach einem der vorhergehenden Ansprüche, **dadurch gekennzeichnet, dass** sich die Teilschlauchgestricke im Übergangsbereich zum Hauptschlauchgestrick teilweise überlappen.

5. Kompressionsgestrick nach einem der vorhergehenden Ansprüche, **dadurch gekennzeichnet, dass** es der Form des zu stützenden Körperteils durch entsprechende Durchmesseränderungen der Schlauchgestricke anatomisch angepasst gestrickt ist.

6. Kompressionsartikel nach einem der vorhergehenden Ansprüche, **dadurch gekennzeichnet, dass** es unter Verwendung mindestens eines elastischen Schussfadens hergestellt ist.

7. Verfahren zur Herstellung eines Kompressionsartikels nach einem der Ansprüche 1 bis 7 auf einer Flachstrickmaschine, **dadurch gekennzeichnet, dass** am Übergang von den Teilschlauchgestricken zum Hauptschlauchgestrick einige der Maschen benachbarter Teilschlauchgestricke auf gemeinsame Nadeln gehängt und in kurzen Zwischenmaschenreihen abgestrickt werden, bevor die Maschenzahl der Teilschlauchgestricke durch Umhängevorgänge und Nadelbettenversatzbewegungen über mehrere Maschenreihen hinweg gemindert werden.

8. Verfahren nach Anspruch 7, **dadurch gekennzeichnet, dass** die Umhängevorgänge und Versatzbewegungen mit Hilfe von Zusatznadelbetten durchgeführt werden.

9. Verfahren nach Anspruch 7 oder 8, **dadurch gekennzeichnet, dass** die Teilschlauchgestricke und das Hauptschlauchgestrick jeweils in einer Rippbindung mit einem durchgehenden Schussfaden hergestellt werden.

10. Verfahren nach einem der Ansprüche 7 bis 9, **dadurch gekennzeichnet, dass** die Rippbindung mit einem unelastischen Faden und einem Elastikfaden hergestellt wird.

11. Verfahren nach einem der Ansprüche 7 bis 10, **dadurch gekennzeichnet, dass** Maschenreihen der Rippbindung ausschließlich mit einem unelastischen Faden hergestellt werden.

12. Verfahren nach Anspruch 11, **dadurch gekennzeichnet, dass** mit den mit dem unelastischen Faden hergestellten Maschenreihen der Schussfaden in das Gestrick eingebunden wird.

## Claims

1. Compression item with a main tubular knit, into which at least two part tubular knits open out, in which the main and part tubular knits are produced seamlessly on a flat knitting machine, **characterised in that** in the area of transition between the part tubular knits and the main tubular knit there is a stitch reduction area extending over several rows of stitches on the part knits.

2. Compression item according to claim 1, **characterised in that** the stitch reductions are arranged at least at a peripheral location of the part tubular knits arranged between the front and back layer of the knit.

3. Compression item according to claim 1 or 2, **characterised in that** the part tubular knits have several stitch reduction locations distributed over their periphery.

4. Compression knit according to one of the previous claims, **characterised in that** the part tubular knits partly overlap in the area of transition into the main tubular knit.

5. Compression knit according to one of the previous claims, **characterised in that** it is knitted so that anatomically it fits the shape of the part of the body to be supported through corresponding changes in diameter of the tubular knits.

6. Compression item according to one of the previous claims, **characterised in that** it is produced using at least one elastic weft thread.

7. Method for producing a compression item according to one of claims 1 to 7 on a flat knitting machine, **characterised in that** at the transition from the part tubular knits to the main tubular knit some of the stitches of adjacent part tubular knits are on common needles and cast off in short rows between stitches, before the number of stitches of the part tubular knits is reduced through cape processes and offsetting movements of the needle beds over several rows of stitches.

8. Method according to claim 7, **characterised in that** the cape processes and offsetting movements are carried out with the help of additional needle beds.

9. Method according to claim 7 or 8, **characterised in that** the part tubular knits and the main tubular knit are produced in a rib weave with a continuous weft thread.

10. Method according to one of claims 7 to 9, **characterised in that** the rib weave is produced with a non-elastic thread and an elastic thread.

11. Method according to one of claims 7 to 10, **characterised in that** rows of stitches of the rib weave are produced exclusively with a non-elastic thread.

12. Method according to claim 11, **characterised in that** the weft thread is incorporated into the knit with the rows of stitches produced with the non-elastic thread.

## Revendications

1. Article de compression avec un tricot tubulaire principal, dans lequel débouchent au moins deux tricots tubulaires partiels, dans lequel le tricot tubulaire principal et les tricots tubulaires partiels sont fabriqués sans couture sur une tricoteuse rectiligne, **caractérisé en ce qu'**est présente, dans la région du passage entre les tricots tubulaires partiels et le tricot tubulaire principal, une zone de diminution de mailles, s'étendant sur plusieurs rangées de mailles, au niveau des tricots partiels.

2. Article de compression selon la revendication 1, **caractérisé en ce que** les diminutions de mailles sont disposées au moins au niveau d'un emplacement périphérique des tricots tubulaires partiels disposé entre une couche de tricot avant et arrière.

3. Article de compression selon la revendication 1 ou 2, **caractérisé en ce que** les tricots tubulaires partiels présentent plusieurs emplacements de diminution de mailles de manière répartie sur leur périphérie.

4. Tricot de compression selon l'une quelconque des revendications précédentes, **caractérisé en ce que** les tricots tubulaires partiels se chevauchent en partie dans la région de transition menant au tricot tubulaire principal.

5. Tricot de compression selon l'une quelconque des revendications précédentes, **caractérisé en ce qu'**il est tricoté avec une adaptation anatomique à la forme de la partie du corps à soutenir par des modifications de diamètre correspondantes des tricots tubulaires.

6. Article de compression selon l'une quelconque des revendications précédentes, **caractérisé en ce qu'**il est fabriqué en utilisant au moins un fil de trame élastique.

7. Procédé pour la fabrication d'un article de compression selon l'une quelconque des revendications 1 à 7 sur une tricoteuse rectiligne, **caractérisé en ce qu'**au niveau de la transition depuis les tricots tubulaires partiels vers le tricot tubulaire principal, certaines des mailles de tricots tubulaires partiels adjacents sont accrochées à des aiguilles communes et sont tricotées en de courtes rangées de mailles intermédiaires avant que le nombre de mailles des tricots tubulaires partiels ne soit diminué par des opérations de transfert et des déplacements décalés de planches d'aiguilles au-delà de plusieurs rangées de mailles.

8. Procédé selon la revendication 7, **caractérisé en ce que** les opérations de transfert et les déplacements décalés sont effectués à l'aide de planches d'aiguilles supplémentaires.

9. Procédé selon la revendication 7 ou 8, **caractérisé en ce que** les tricots tubulaires partiels et le tricot tubulaire principal sont fabriqués respectivement en un reps avec un fil de trame continu.

10. Procédé selon l'une quelconque des revendications 7 à 9, **caractérisé en ce que** le reps est fabriqué avec un fil non élastique et un fil élastique.

11. Procédé selon l'une quelconque des revendications 7 à 10, **caractérisé en ce que** des rangées de mailles du reps sont fabriquées exclusivement avec un fil non élastique.

12. Procédé selon la revendication 11, **caractérisé en ce qu'**avec les rangées de mailles des fils de trame fabriquées avec le fil non élastique, le fil de trame est intégré dans le tricot.
